# EUROPÄISCHE PATENTSCHRIFT

(11) **EP 1 419 748 B1**
(45) Veröffentlichungstag und Bekanntmachung des Hinweises auf die Patenterteilung: **19.07.2006**
(21) Anmeldenummer: 02405992.5
(22) Anmeldetag: 18.11.2002
(51) Int. Cl.: A61F 2/16

(54) **Linseneinführvorrichtung mit Fingerring**
Lens injector with finger ring
Dispositif d'injection de lentille avec bague

(43) Veröffentlichungstag der Anmeldung: 19.05.2004
(73) Patentinhaber: Anton Meyer & Co.AG, 2560 Nidau (CH); Asico LLC, Westmont, IL 60559 (US)
(72) Erfinder: Meyer, Rolf, 2562 Port (CH)
(74) Vertreter: Clerc, Natalia

(56) Entgegenhaltungen:
- EP-A- 0 477 466
- US-A- 5 643 276
- US-A- 5 728 102
- US-B1- 6 179 843

## Beschreibung

### Technisches Gebiet

Die Erfindung betrifft eine handbetätigbare Vorrichtung zur Einführung einer Linse in ein Auge gemäss Oberbegriff des Patentanspruchs 1.

### Stand der Technik

In der heutigen Augenchirurgie zählt die Ersetzung einer trüben natürlichen Augenlinse durch eine künstliche Linse zu den Routineeingriffen. Dennoch erfordert dieser Eingriff vom Chirurgen eine überaus sichere Hand und eine grosse Übung.

Es sind deshalb handbetätigbare Vorrichtungen, sogenannte Injektoren, bekannt, welche eine gewisse Führung beim Einsetzen der künstlichen Linse ermöglichen. Im allgemeinen weisen diese bekannten Injektoren einen Griffkörper und einem im Griffkörper über ein Gewinde verschiebbaren Kolben auf. Im vorderen Bereich des Griffkörpers ist einen Linsenaufnahme vorhanden, in welche eine einzuführende künstliche Linse eingelegt wird. Durch Drehen des Kolbens lässt sich nun diese Linse durch eine vordere Öffnung der Linsenaufnahme durchschieben, wobei die Linse dabei gegebenenfalls gefaltet wird. Durch weiteres Vorschieben der Linse wird sie im gefalteten Zustand ins Auge eingebracht. Einen derartigen Injektor offenbart beispielsweise US-A-5'643'276.

Ferner ist in der nachveröffentlichten europäischen Patentanmeldung EP-A-1287791 der Anmelderin ein handbetätigbarer Injektor beschrieben, welcher eine einhändige Bedienung erlaubt und trotzdem eine sichere Führung des Kolbens garantiert. Bei diesem Injektor ist der Kolben in einer Kugellagerbüchse lateral verschiebbar gelagert. Die präzise laterale Verschiebung ist durch eine Führungsnut gewährleistet, in welche ein im Griffkörper angeordnetes Führungselement eingreift. In einer Ausführungsform ist der Kolben drehfest lateral verschiebbar. In einer anderen Ausführungsform weist der Kolben einen Wendel auf, um am Schluss der Einführbewegung noch eine Drehung des Kolbens zu ermöglichen. Dies erleichtert die Einführung der künstlichen Linse, insbesondere deren Entfaltung im Auge. Diese Drehung ist jedoch von der Gestaltung des Injektors, insbesondere des Wendels vorbestimmt.

US-A-5'728'102 offenbart einen Injektor, in dessen rohrförmigem Griffkörper ein Kolben drehfest geführt ist. Am hinteren Kolbenende ist ein Betätigungselement drehbar befestigt.

### Darstellung der Erfindung

Es ist Aufgabe der Erfindung, eine Vorrichtung der eingangs genannten Art zu schaffen, bei welcher der Chirurg den Zeitpunkt und das Ausmass der Drehbewegung selber bestimmen kann, ohne jedoch die sichere Führung und stabile Haltung der Vorrichtung zu beeinträchtigen.

Diese Aufgabe löst eine Vorrichtung mit den Merkmalen des Patentanspruchs 1.

Die erfindungsgemässe Vorrichtung weist einen in einem Griffkörper verschiebbaren Kolben mit einem Betätigungsmittel auf. Erfindungsgemäss ist das Betätigungsmittel relativ zum Kolben drehbar, und das Betätigungsmittel umfasst einen Fingerring, insbesondere für einen Daumen.

Dadurch ist es dem Chirurgen möglich, mit einer ersten Hand den Injektor zu halten und die Linse sicher geführt in das Auge einzuführen. Will er zu irgend einem Zeitpunkt der Operation, insbesondere wenn die Linse in das Auge eingeführt ist, die Linse drehen, so lässt sich dies durch Drehung des Kolbens beziehungsweise des Griffkörpers erreichen, ohne dass das Betätigungsmittel mitdreht. Die Hand beziehungsweise der Daumen dieser Hand muss die Lage nicht ändern. Dem Chirurgen ist es somit möglich, eine Drehung von Hand, vorzugsweise mit der anderen Hand, auszuführen, ohne die stabile Haltung des Injektors zu gefährden.

In einer einfachen Ausführungsform umfasst das Betätigungsmittel einen Kolbenkopf mit einer geeignet geformten Stirnfläche zur Betätigung des Kolbens.

Weitere vorteilhafte Ausführungsformen gehen aus den abhängigen Patentansprüchen hervor.

### Kurze Beschreibung der Zeichnung

Im folgenden wird der Erfindungsgegenstand anhand eines bevorzugten Ausführungsbeispiels, welches in den beiliegenden Zeichnung dargestellt ist, erläutert. Es zeigen:
- Figur 1: einen Querschnitt durch einen erfindungsgemässen Injektor;
- Figur 2: eine Explosionsdarstellung eines Teils eines Kolbens mit einem erfindungsgemässen Betätigungsmittel und
- Figur 3: eine perspektivische Darstellung des erfindungsgemässen Betätigungsmittels mit einem Teil des Kolbens in zwei Drehstellungen.

### Wege zur Ausführung der Erfindung

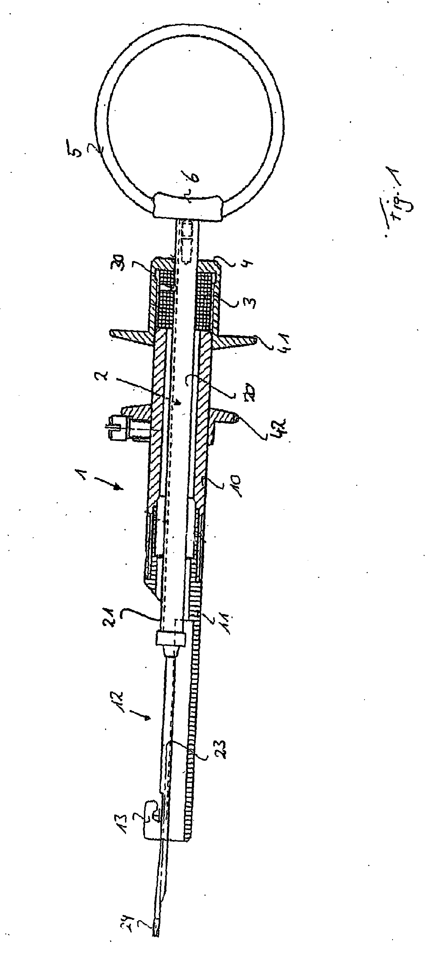

In Figur 1 ist eine erfindungsgemässe handbetätigbare Vorrichtung zur Einführung einer Linse in ein Auge, ein sogenannter Injektor, dargestellt.

Er ist im wesentlichen gleich aufgebaut wie der in der eingangs erwähnten europäischen Patentanmeldung EP-A-1287791.

Der Injektor weist einen Injektorkörper oder Griffkörper 1 auf, in welchem ein Kolben 2 verschiebbar gelagert ist. Sowohl Griffkörper 1 wie auch Kolben 2 sind vorzugsweise aus Metall, insbesondere aus Titan, gefertigt.

Der Griffkörper 1 weist eine Hülse 10 auf, welche an ihrem vorderen Ende in ein Griffvorderteil 11 übergeht. Das Griffvorderteil 11 weist an seinem vorderen, der Hülse 10 abgewandten Ende eine Linsenaufnahme 13 auf, wobei eine entsprechende künstliche Linse über ein hinter der Linsenaufnahme 13 angeordnetes Einführfenster 12 eingelegt wird.

Der Kolben 2 weist eine Kolbenstange 20 auf, welche in eine Kolbennadel 23 mit einer Kolbenspitze 24 übergeht. Die Kolbennadel 23 ist vorzugsweise lösbar mit der Kolbenstange 20 verbunden, so dass je nach Art der Linse eine neue Nadel verwendet werden kann. Zudem lässt sie sich auch als Einwegnadel verwenden. Die Kolbenstange 20 kann ein- oder mehrteilig ausgebildet sein.

Der Kolben 2 beziehungsweise seine Kolbenstange 20 ist in der Hülse 10 geführt verschiebbar angeordnet. Im hier dargestellten Beispiel ist hierfür eine Kugellagerbüchse 3 vorhanden, welche lagefixiert in der Hülse 10 angeordnet ist. Vorzugsweise ist sie im hinteren, der Kolbenspitze 23 abgewandten Ende der Hülse 10 angeordnet. Vorzugsweise weist die Kolbenstange 20 eine Führungsnut 21 auf und in der Hülse 10, in der Kugellagerbüchse 3 oder einem anderen fest mit der Hülse 10 verbundenen Teil ist ein Führungselement 30, vorzugsweise eine gefederte Metall- oder Kunststoffkugel, angeordnet. Das Führungselement 30 greift in die Führungsnut 21 ein und ermöglicht so eine verbesserte gezielte Führung des Kolbens 2 bei seiner Verschiebung relativ zum Griffkörper 1. Die Führungsnut 21 ist dabei vorzugsweise geradlinig ausgebildet so dass der Kolben 2 drehfest longitudinal (lateral) verschiebbar ist.

Wie in Figur 1 ersichtlich ist, ist der Griffkörper 1 vorzugsweise mit einer Abschlusskappe 4 verschlossen, welche vom Kolben 2 durchsetzt ist. An der Verschlusskappe 4 und/oder am Griffkörper sind vorzugsweise Flansche 41, 42 angeordnet, welche wie bei einer Spritze als Anschlag für die Finger des Chirurgen dienen. Vorzugsweise ist der zweite Flansch 42 ein separates Element, welches über ein Befestigungsmittel, hier eine Schraube, lösbar an der Hülse 10 befestigbar ist. Dadurch lässt sich der zweite Flansch 42 entlang der Hülse 10 verschieben, so dass sich der Abstand zwischen den zwei Flanschen 41, 42 entsprechend der Fingerdicke des Chirurgen anpassen lässt.

Am hinteren, der Kolbenspitze 24 abgewandten Ende des Kolbens 2 ist ein Betätigungsmittel zur Verschiebung des Kolbens 2 relativ zum Griffkörper 1 angeordnet. Dieses Betätigungsmittel ist relativ zum Kolben 2 drehbar, so dass eine Drehung des Griffkörpers 1 zwar eine Drehung des Kolbens 2, nicht aber eine Drehung des Betätigungsmittels zur Folge hat. Vorzugsweise ist es um 360° drehbar, es ist jedoch auch möglich lediglich eine Schwenkung zuzulassen.

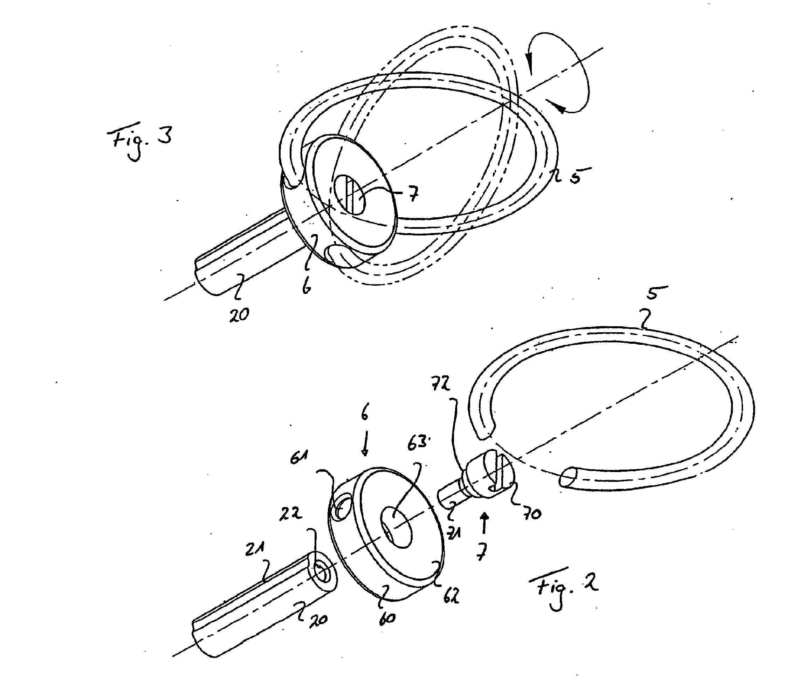

Im hier dargestellten Beispiel umfasst das Betätigungsmittel einen Kolbenkopf 6 und einen Fingerring 5, insbesondere einen Daumenring. Diese sind am besten in den Figuren 2 und 3 erkennbar.

Der Fingerring 5 ist lagefixiert am Kolbenkopf 6 angeordnet. Der Kolbenkopf 6 ist als flacher Zylinder ausgebildet, wobei er eine zentrale Durchgangsöffnung 63 aufweist. In der Durchgangsöffnung 63 ist eine Schulter vorhanden. Der Kolbenkopf 6 ist mittels einer Blockierschraube 7 mit dem hinteren Ende der Kolbenstange 20 verbunden. Die Kolbenstange 20 weist hierfür eine Gewindebohrung 22 auf, in welchem ein Schraubengewinde 71 der Blockierschraube 7 eingreift. Zwischen Schraubenkopf 70 und Schraubengewinde 71 weist die Blockierschraube 7 eine Schulter 72 auf, welche in axialer Richtung etwas länger ausgebildet ist als diejenige des Kolbenkopfs 6. Zudem ist der Schraubenkopf 70 im Durchmesser etwas kleiner als die Durchgangsöffnung 63. Dadurch lässt sich der Kolbenkopf 6 relativ zur Kolbenstange 20 und zur Blockierschraube 7 drehen. Die freie Stirnfläche 62 des Kolbenkopfs 6 oder Druckknopfs dient als Druckfläche für den Finger, insbesondere den Daumen des Chirurgen, um den Kolben durch den Injektor zu stossen. Die Stirnfläche 62 kann flach ausgebildet sein oder eine Mulde zur besseren Aufnahme des Daumens aufweisen.

In der Mantelfläche 60 des Kolbenkopfs 6 sind zwei Einstecköffnungen 61 vorhanden. Diese sind vorzugsweise diametral entgegengesetzt angeordnet. In diese Einstecköffnungen lässt sich der offene Fingerring 5 einstecken, wie in Figur 2 erkennbar ist. Diese Anordnung weist den Vorteil auf, dass der Fingerring 5 entsprechend der Fingerdicke des Chirurgen ausgewählt werden kann und auf einfache Weise auf den Injektor montiert werden kann.

Zudem weist das oben beschriebene Betätigungsmittel den Vorteil auf, dass es aus relativ wenigen Teilen besteht, welche sich auf einfache Art und Weise montieren und demontieren lassen, so dass das Betätigungsmittel sich optimal reinigen lässt.

Das erfindungsgemässe drehbare Betätigungsmittel lässt sich auch in anderen handbetätigbaren Injektoren einsetzen. Bevorzugt sind dabei alle Injektoren, welche eine einhändige Bedienung erlauben.

Die erfindungsgemässe Vorrichtung ermöglicht somit eine Drehung des Injektors um einen beliebigen Winkel, ohne dass die Position der den Kolben drückenden Hand, insbesondere die Daumenstellung, wesentlich geändert werden muss.

### Bezugszeichenliste

- 1: Griffkörper
- 10: Hülse
- 11: Griffvorderteil
- 12: Einführfenster
- 13: Linsenaufnahme
- 2: Kolben
- 20: Kolbenstange
- 21: Führungsnut
- 22: Gewindebohrung
- 23: Kolbennadel
- 24: Kolbenspitze
- 3: Kugellagerbüchse
- 30: Führungselement
- 4: Abschlusskappe
- 41: erster Flansch
- 42: zweiter Flansch
- 5: Fingerring
- 6: Kolbenkopf
- 60: Mantelfläche
- 61: Einstecköffnung
- 62: Stirnfläche
- 63: Durchgangsöffnung
- 7: Blockierschraube
- 70: Schraubenkopf
- 71: Schraubengewinde
- 72: Schulter

## Patentansprüche

1. Handbetätigbare Vorrichtung zur Einführung einer Linse in ein Auge, wobei die Vorrichtung einen Griffkörper (1) mit einer Linsenaufnahme (13) zur Aufnahme einer Linse und einen im Griffkörper (1) verschiebbar angeordneten Kolben (2) zur geführten Einführung der Linse in das Auge aufweist, wobei am Kolben (2) ein Betätigungsmittel (5, 6) zur Verschiebung des Kolbens (2) relativ zum Griffkörper (1) angeordnet ist, und wobei das Betätigungsmittel relativ zum Kolben (2) drehbar ist, **dadurch gekennzeichnet, dass** das Betätigungsmittel (5, 6) einen Fingerring umfasst.

2. Vorrichtung nach Anspruch 1, **dadurch gekennzeichnet, dass** das Betätigungsmittel einen Kolbenkopf (6) aufweist, welcher am Kolben (2) drehbar befestigt ist.

3. Vorrichtung nach Anspruch 2, **dadurch gekennzeichnet, dass** der Fingerring (5) lagefixiert am Kolbenkopf (6) befestigbar ist.

4. Vorrichtung nach Anspruch 1, **dadurch gekennzeichnet, dass** der Fingerring (5) einen Durchmesser in einer Grösse aufweist, welcher zur Aufnahme eines Daumens eines Chirurgen geeignet ist.

5. Vorrichtung nach Anspruch 2, **dadurch gekennzeichnet, dass** der Kolbenkopf (6) mittels einer Blockierschraube (7) drehbar mit dem Kolben (2) verbunden ist, wobei der Kolben (2) eine Kolbenstange (20) mit einer Gewindebohrung (22) aufweist und die Blockierschraube (7) in dieser Gewindebohrung (22) eingeschraubt ist.

6. Vorrichtung nach einem der Ansprüche 1 bis 5, **dadurch gekennzeichnet, dass** der Kolben (2) relativ zum Griffkörper (1) longitudinal und drehfest verschiebbar ist.

7. Vorrichtung nach einem der Ansprüche 1 bis 6, **dadurch gekennzeichnet, dass** der Kolben (2) eine Führungsnut (21) aufweist und dass im Griffkörper (1) mindestens ein Führungselement (36) angeordnet ist, welches in die Führungsnut (21) eingreift.

8. Vorrichtung nach einem der Ansprüche 1 bis 7, **dadurch gekennzeichnet, dass** der Kolben (2) in einer Kugellagerbüchse (3) geführt verschiebbar ist, welche drehfest im Griffkörper (1) angeordnet ist.

## Claims

1. A hand-operated device for inserting a lens into an eye, said device having a grip body (1) with a lens holder (13) for receiving a lens, and a plunger (2) arranged displaceably in the grip body (1) for guided insertion of the lens into the eye, an actuating means (5, 6) for displacing the plunger (2) relative to the grip body (1) being arranged on the plunger (2), the actuating means being rotatable relative to the plunger (2), **characterized in that** the actuating means (5, 6) comprises a finger ring.

2. The device as claimed in claim 1, **characterized in that** the actuating means comprises a plunger head (6) which is rotatably mounted on the plunger (2).

3. The device as claimed in claim 2, **characterized in that** the finger ring (5) can be secured fixed in position on the plunger head (6).

4. The device as claimed in claim 1, **characterized in that** the finger ring (5) has a diameter of such a size that it is adapted for receiving a surgeon's thumb.

5. The device as claimed in claim 2, **characterized in that** the plunger head (6) is connected rotatably to the plunger (2) by means of a locking screw (7), the plunger (2) having a plunger rod (20) with a threaded bore (22), and the locking screw (7) being screwed into this threaded bore (22).

6. The device as claimed in one of claims 1 to 5, **characterized in that** the plunger (2) is longitudinally displaceable relative to the grip body (1) but fixed in terms of rotation.

7. The device as claimed in one of claims 1 to 6, **characterized in that** the plunger (2) has a guide groove (21), and that at least one guide element (36) is arranged in the grip body (1) and engages in the guide groove (21).

8. The device as claimed in one of claims 1 to 7, **characterized in that** the plunger (2) is displaceable in a guided manner in a ball-bearing bush (3) which is arranged in a rotationally fixed manner in the grip body (1).

## Revendications

1. Dispositif manuel d'injection d'une lentille dans un oeil, dans lequel le dispositif présente un corps de préhension (1) avec un logement de lentille (13) pour recevoir une lentille et un piston (2) disposé à coulissement dans le corps de préhension (1) pour l'injection guidée de la lentille dans l'oeil, un moyen d'actionnement (5, 6) étant disposé sur le piston (2) pour faire coulisser le piston (2) par rapport au corps de préhension (1), et le moyen d'actionnement pouvant tourner par rapport au piston (2), **caractérisé en ce que** le moyen d'actionnement (5, 6) comprend une bague.

2. Dispositif selon la revendication 1, **caractérisé en ce que** le moyen d'actionnement présente une tête de piston (6) qui est fixée de manière rotative sur le piston (2).

3. Dispositif selon la revendication 2, **caractérisé en ce que** la bague (5) peut être fixée de manière fixe en position sur la tête de piston (6).

4. Dispositif selon la revendication 1, **caractérisé en ce que** la bague (5) présente un diamètre ayant une taille appropriée pour recevoir le pouce d'un chirurgien.

5. Dispositif selon la revendication 2, **caractérisé en ce que** la tête de piston (6) est connectée de manière rotative au piston (2) au moyen d'une vis de blocage (7), le piston (2) présentant une tige de piston (20) avec un alésage fileté (22) et la vis de blocage (7) étant vissée dans cet alésage fileté (22).

6. Dispositif selon l'une quelconque des revendications 1 à 5, **caractérisé en ce que** le piston (2) peut coulisser par rapport au corps de préhension (1) longitudinalement et de manière fixe en rotation.

7. Dispositif selon l'une quelconque des revendications 1 à 6, **caractérisé en ce que** le piston (2) présente une rainure de guidage (21) et **en ce qu'**au moins un élément de guidage (36) est disposé dans le corps de préhension (1), lequel vient en prise dans la rainure de guidage (21).

8. Dispositif selon l'une quelconque des revendications 1 à 7, **caractérisé en ce que** le piston (2) peut coulisser de manière guidée dans une douille de roulement à bille (3), qui est disposée de manière fixe en rotation dans le corps de préhension (1).
